# EUROPEAN PATENT SPECIFICATION

(11) **EP 1 509 121 B1**
(45) Date of publication and mention of the grant of the patent: **26.09.2012**
(21) Application number: 03722819.4
(22) Date of filing: 06.05.2003
(51) Int. Cl.: A61B 3/032, A61B 3/08, A61B 3/024

(54) **OCULAR DISPLAY APPARATUS FOR TREATMENT OF OCULAR DISORDERS**
BILDANZEIGEVORRICHTUNG ZUR BEHANDLUNG VON OKULAREN STÖRUNGEN
APPAREIL DE VISUALISATION OCULAIRE POUR LE TRAITEMENT DE TROUBLES OCULAIRES

(30) Priority: 04.05.2002 GB 0210288
(43) Date of publication of application: 02.03.2005
(73) Proprietor: The University of Nottingham, Nottingham NG7 2RD (GB)
(72) Inventor: SMART, Paula Virtual Reality App. Res.Team(VIRART), University Park Nottingham NG7 (GB); COBB, Sue Virtual Reality App. Res. Team(VIRART), Nottingham NG7 2RD (GB); MOODY, Amanda Virt. Reality Ap. Res.T.(VIRART), Nottingham NG7 2RD (GB); EASTGATE, Richard Virt. Reality App. Res.(VIRART), Nottingham NG7 2RD (GB); GRIFFITHS, Gareth Virtual Reality App. Res.(VIRART), Nottingham NG7 2RD (GB); BUTLER, Tom c/o James Paget Hospital, Gorleston Great Yarmouth Norfolk NR316LA (GB); COMAISH, Ian Orthoptic Department QMC, Nottingham NG7 2UH (GB); HAWORTH, Stephen Orthoptic Department QMC, Nottingham NG7 2UH (GB); GREGSON, Richard Orthoptic Department QMC, Nottingham NG7 2UH (GB); ASH, Isabel Orthoptic Department QMC, Nottingham NG7 2UH (GB); BROWN, Sarah Orthoptic Department QMC, Nottingham NG7 2UH (GB)
(74) Representative: Lawrence, John
(86) International application number: PCT/GB2003/001909
(87) International publication number: WO 2003/092482

(56) References cited:
- EP-A- 0 830 839
- EP-A- 1 082 939
- US-A- 5 309 185
- US-A- 5 920 374
- US-B1- 6 206 522

## Description

This invention relates to ocular display apparatus which may be used to assess visual function and ocular motility, and to treat disorders thereof. In particular, the invention relates to ocular display apparatus which presents different, but visually related, images or environments to each eye, in which there is at least one moving object. The apparatus may be configured as an interactive binocular display apparatus. It also relates to methods of assessment of ocular disorders and to their treatment.

There are a number of common eye conditions where a person's eyes fail to work correctly in combination with each other. These conditions can result in blurred or double vision, or the absence of true stereovision. These conditions are often the result of physical problems, i.e. strabismus, commonly known as a squint, which can result in amblyopia, commonly known as a 'lazy eye'.

This invention has particular relevance to the assessment and treatment of amblyopia or 'lazy eye', however it is applicable to the study and treatment of many eye conditions, and possibly vision/co-ordination related brain problems, or neurological pathway developmental problems.

Amblyopia is a condition of the visual system in which one eye fails to develop a normal level of visual acuity during the developmental period for vision in the absence of ocular disease. Amblyopia can occur in subjects who are strabismic (have a squint), or are mixed amblyopes (have a squint and anisometropia - such that both eyes have different refractive errors, leaving one eye defocused), or have a cataract which can result in amblyopia due to stimulus deprivation. The poor vision resulting from amblyopia cannot be corrected by optical means. Amblyopia is a common condition of childhood affecting perhaps as many as 2-3 percent of the population, and can carry over into adult life if left untreated. Whilst most people can manage with a lazy eye, they may well have reduced or no binocular function and this may compromise their ability to perform certain complex tasks, such as flying an aeroplane or driving a train. Furthermore, persons with one amblyopic eye who suffer injury to their 'normal' eye account for a small, but significant, number of people on the blind register.

Whilst many treatments for amblyopia have been attempted the most common remains the use of a patch, which has been advocated for decades, indeed use is documented as early as the eighteenth century. More specifically, the non-amblyopic eye is covered with a patch for prescribed periods of time daily, (e.g. hours a day) perhaps for several months, and even years. The patching of the non-amblyopic eye forces the wearer to use and hence stimulate the amblyopic eye. A major drawback with the use of the patch is subject compliance. That is, ensuring the subject wears the patch for the prescribed time periods. Most subjects are children many of whom do not want to wear the patch as their vision is much poorer when using only the lazy eye, or they are teased when wearing the patch and so refuse to wear it. Non-compliance is a major cause of failure of the patching technique in treating children with amblyopia. It is thought that children with amblyopia are best treated before the age of eight years.

Searches performed with hindsight have identified the following documents: GB 2 353 869 to a synaptophore using computer display to measure a squint; EP 0 830 839 to a binocular view function apparatus and inspecting method; and US 4 756 305 to an eye training device.

EP 1 082 939 discloses a synoptophore for the assessment of strabismus patients.

The invention provides an ocular display apparatus for the treatment of amblyopia, the apparatus having image presentation means adapted to display a first image to one eye only of a subject, and a second, different, image to the subject's other eye only, the first and second images being presented to the subject so that they perceive a composite image, characterised in that the first image comprises a first sub-image and the second image comprises a second sub-image, the sub-images when superimposed produce a composite image, wherein
the first sub-image and the second sub-image each have a common component superimposable as seen by a subject so as to be seen as only one sub-image by the subject, and in which one of the sub-images also has at least one additional feature not present in the other sub-image,
the additional feature arranged to be presented to the eye that performs worst/badly in order to treat amblyopia; and
wherein at least one of the first or second images includes a moving object in addition to stationary objects.

Preferably, the subject is visually immersed in the displayed images.

By 'visually immersed' we mean that the subject sees substantially only the displayed images. The subject has no significant peripheral vision outside the displayed images, and so is not readily visually distracted by incidental movement in their peripheral vision. This is particularly important if children are the subjects as they are easily distracted.

The apparatus may alternatively be termed an image display device or image production device.

Preferably the images are computer generated and may be presented to the subject using a spilt screen or two separate screens, configured as a 'table top' or hand held viewer, or a headset. The images may be perceived as two dimensional (2D), three dimensional (3D) or virtual reality.

In at least one embodiment of the present invention the apparatus uses virtual reality technology.

The apparatus may comprise image presentation means configured as one or more screens. Preferably, the image presentation means are configured as first and second screens on which the first and second images are respectively displayed, which includes a barrier means adapted to allow each eye of a subject to see a respective first or second screen, and to prevent each eye of the subject from seeing the other of the first or second screens. Alternatively, the images may be presented on discrete parts of a single screen, each eye being able to see only a respective one of the images.

Alternatively, the image presentation means may include a projector adapted to project the first and second images. First and second screens may be provided on which the first and second images are respectively projected.

Alternatively, or additionally, the image may be generated in some other way, for example using a CRT screen, a plasma screen or an LCD etc.

The image may be generated on a pixellated screen in which the pixels are individually electronically addressed, for example one which employs a raster array. This may allow a practically infinite number of images to be displayed on the screen and allows for the presentation of movement of an object on the screen which appears smooth to the subject/viewer (i.e. like a video, rather than like jumping between illuminated pictures).

The device may comprise a stereoscopic viewer which displays a different image to each eye. The system can be used to present a range of static or dynamic, 2D or 3D images to the subject.

As the subject is visually immersed their whole visual field can be studied, as opposed to studying only their central vision and not their peripheral vision.

Preferably, the movement in at least one of the first or second images is perceived by the subject to be smooth/continuous.

By a moving object it will be understood that this includes a varying or changing visual object within the image or scene, such as, hands on a clock face, characters moving in a cartoon, or a racing car travelling around a track etc. This movement, or perception of movement, can be achieved, for example, by displaying a sequence of slightly different images in time, provided that the time between the images is short enough that the viewer perceives a moving object in the image.

Each eye may be presented with a different image simultaneously, or alternatively, the images may be presented in quick succession, alternately, so as effectively to be simultaneously displayed, as perceived by the subject.

Indeed, the first and second images may differ only in the colour, contrast, intensity or focus of at least part of one of the images. Alternatively the images may be very different and have no features in common.

The movement may be in the whole visual field of the subject, or it may just be in the central field of view or it may be just in the peripheral field of view. The field of view where the movement occurs may change. Typically, if the aim is to stimulate a lazy eye the movement will be in the centre of the image displayed to the lazy eye. If the intention is to test or exercise peripheral characteristics on a subjects vision the movement may be in the peripheral field of view of the subject.

The peripheral image may be shown to one eye and the central image may be shown to the other eye. This allows stimulation of the central vision. This is important in the treatment of amblyopia, where the 'lazy' eye may be shown the more stimulating central image, and the 'better' eye is shown the peripheral image, thereby forcing the lazy eye to work harder.

Each eye may be shown an image with movements in different parts of the field of view. What is moved in the image, and the extent to which it is moved, may be varied dependent on the subject's condition, e.g. their degree of amblyopia, and to keep the subject constantly visually and cognitively engaged.

Preferably the images are displayed to the subject, and viewed by the subject, in full colour.

The images are preferably not viewed via prisms as the use of prisms introduced chromatic aberrations and degrades the visual image, and full colour cannot be viewed properly. Introducing a prism, or any other optical element, may cause significant chromatic aberration.

Preferably the images are presented to the subject along the direction of the visual axis each eye, taking into account any squint the subject may have. That is, the image may be presented to the subject at an angle equal to that of the subject's angle of squint. Each eye of a subject may have their respective images presented as different angles (i.e. if each eye has a visual axis in a different direction).

The subject's perception of the image will be determined by their ocular characteristics (ie whether the subject has a squint, ocular torsion or other visual disorder). The position of the first and second images may have to be adjusted from one subject to the next in order that each subject perceives the same composite image. The aim of many embodiments is that all subjects perceive the same composite image even though the position of the first and second images may be different.

Preferably, the subject must use both eyes in order to view the composite image. If both eyes are not used together the user will not see the whole composite image. This apparatus can be used to encourage use of a lazy eye, say be presenting more stimulating images to the lazy eye and encouraging it to work harder, in a more like real life environment than patching the eye.

The apparatus may be configured such that at least one moving object is present only to one eye. Alternatively, a different moving object may be presented seperately to each eye.

Preferably, the apparatus is adapted to display first and second images that are related to each other.

Preferably, the first image has at least one additional feature not present in the second image. Alternatively, the first image may have at least one first additional feature not present in the second image, and the second image may have at least one second additional feature not present in the first image.

Preferably the overall, combined image is recognisable as a known thing by the subject, for example as a known article, event, scenario, etc.

The apparatus allows a subject to see a common-to-both sub-images component aligned/superimposed as a single feature of the combined image, and also the additional feature. The additional feature may be presented to the eye that performs worst/badly. The additional feature may comprise an eye-catching, interesting feature, possibly the focus/centre of attention of the combined image. The common-to-both sub-images may be dull, uninteresting features (in comparison to the additional feature).

Preferably both the first and second images include a moving (ie dynamic) object, such that the image display changes over time.

The apparatus may also be adapted to cause said first image to have stationary objects and said second image to have at least one moving object. The apparatus may have a subject-manipulatable object-control, which is adapted to enable control of the movement of at least one of the moving objects relative to the stationary objects.

Apparatus may also be provided which may be adapted to cause said first image to have at least one first moving object and said second image to have least one second moving object. The apparatus may have a subject or operator-manipulatable object control adapted to enable the movement of at least one of, or both of, the first and/or second moving objects to be controlled. The operator may not be the subject - they could be a clinician, orthoptist or technician.

Preferably the moving object or objects which can be manipulated are controlled by the operator using a joystick, paddle, hand held control device, touch screen, keyboard or other appropriate device. The ability to manipulate objects allows user interaction with the image. This interaction may be achieved by user participation in a game, in which the user controls the movement of at least one of the movable objects. Movement of objects may also be under the control of computer software.

Subject interaction or participation requires both eyes to be used together. The active searching of the visual image or scene by each eye independently in order to fuse the images stimulates binocular brain activity. The use of both eyes together in an interactive visual environment can be used as an exercise or treatment for amblyopia, by stimulating the uses of a lazy eye. Absent the requirement that both eyes are used together in order to see the complete composite image, amblyopia sufferers are inclined to view an image with only their good eye.

The apparatus may comprise an operator display adapted to display to an operator the images seen by the subject, or representations of those images, or an indication of the identity of those images, and/or an indication of the type or degree of eye disorder present in the subject.

This enables the operator to know what the subject is seeing, and possibly to ask questions, to establish what is perceived by the subject. Such subject-feedback may be provided without an operator asking questions - the machine may ask them of the subject, either aurally or visually.

Preferably the apparatus may comprise image manipulation means adapted to enable the first and second images to be manipulated to present them so that a subject perceives the intended composite image. The manipulation means may be provided to the subject or to the operator, or both. A manipulation monitor may be provided adapted to provide information on the type and degree of manipulation of the images required to enable the subject to perceive the intended composite image. The manipulation monitor may provide this information to the operator, possibly in real time as the subject uses the apparatus. The manipulation monitor may comprise a display screen, possibly of a computer (e.g. a PC, or portable/laptop/ palmtop computer).

The apparatus may also comprise a monitor adapted to present said information on a results display. The results may also be provided as paper print-out generated by the apparatus.

Image movement means may be provided adapted to cause relative movement between the first and second images so as to enable their relative positions and/or orientations to be adjusted.

One or more adjustments may be performable from the list:-
(i) moving the first and/or second image horizontally;
(ii) torsional movement of the first and/or second image;
(iii) transposing the positions of the first and second images;
(iv) moving the first and/or second image vertically.

The image movement means may allow a first alignment portion of the first image to be aligned, as seen by the subject, with a second alignment portion of the second image. In order for a subject to perceive the intended composite image, these alignment portions must be perceived by the subject as aligned.

The first and second alignment portions may be provided on a common component and a said additional feature respectively. Thus, a subject may see a desired, recognisably aligned configuration of something presented to one eye only and something presented to both eyes.

Alternatively, the alignment portions may be presented separately to each eye. The user may have to align something seen only with one eye with something seen only by the other eye: that is to say that the first and second alignment portions may be provided on first and second additional features respectively. Thus, a subject may see a desired, recognisably aligned configuration of something presented to one eye only with another thing presented to the other eye only.

The first and second images may each contain a first and second object with alignment portions respectively, in order for the subject to perceive the intended composite image the alignment portions must also be perceived to be aligned.

Image movement means allow the first and/or second images to be moved until the subject perceives the first alignment portion of the first image to be aligned with a second alignment portion of the second image.

Preferably, the apparatus may be configured to allow egocentric (first person) movement, so that it appears to the subject that they are actually moving within the displayed image.

The images presented may be adapted to produce images which are perceived by the subject as 2D, 3D or virtual reality (VR). In traditional VR each eye is presented with the same image from a different perspective. However, in this invention each eye is presented with a different image, that is, the content of each image is not the same.

Preferably the apparatus comprises a computer memory having a library of images and a computer processor adapted to retrieve images and present them to a subject. The apparatus may comprise a user-operable image selector adapted to enable different images to be presented to the subject.

The apparatus may be adapted to present a composite image that presents a game, or viewable performance (for example, a film), or subject-interactive test to the subject.

The playing of a game, for example, requires the interactive involvement of the subject with the images displayed.

Preferably, the interaction is real-time, that is, the subject observes an immediate response to their action.

The apparatus may also be adapted to provide information indicative of (a) the amount and type of corrective movement of images required to enable a subject to see an intended composite image, or (b) information relating to vision defects of a subject, for a plurality of eye disorders, at least one, or some, of which are from the list:-
(1) amblyopia
(2) diplopia
(3) squint
(4) ocular torsion.

The apparatus may also be adapted to measure visual function, more specifically, to measure at least one of:-
(1) visual acuity;
(2) 'crowding' effect;
(3) visual perception time;
(4) a squint in primary position (straight ahead) or ocular misalignment;
(5) a squint in nine (or several) positions of gaze;
(6) ocular torsion;
(7) eye movements;
(8) area of binocular single vision;
(9) the limit of eye movements;
(10) the density of suppression;
(11) the area of suppression;
(12) head posture;
(13) saccades (rapid eye movements);
(14) post-operative diplopia;
(15) binocular visual acuity;
(16) presence of binocular single vision;
(17) the fusion range;
(18) 3-D vision/stereo-acuity/stereopsis;
(19) distance stereopsis; and/or
(20) paediatric visual field.

The apparatus may also be capable of performing 2, 3, 4, 5, or more of items (1) to (20).

The apparatus may be adapted to measure visual function and to present a game, viewable performance or subject-interactive test to the subject.

Preferably the subject or operator can readily switch between test, watch and game modes. The ability of a single piece of apparatus to perform multiple modes of operation has the advantage of providing a versatile piece of apparatus which can perform many different functions, which occupies less floor space and costs less than having a different device for each function.

The apparatus may be portable. By "portable" we mean carriable by single 80kg person without undue difficulty, preferably (but not necessarily) using one hand.

Preferably, the apparatus is also provided with an eye-direction detector; which may comprise an eye monitor adapted to determine the direction in which one, or both, eyes are looking (that is to say, to monitor eye movement).

The apparatus may be adapted to produce three-dimensional images. These are useful in assessing convergence and stereopsis and can be useful in stimulating visual interest.

Preferably the ocular display apparatus includes a computer controller, such as a microprocessor, or a PC, configured to control the images displayed to the subject. A qualified practitioner, such an orthoptist, or a technician may operate the computer controller, or indeed it may be that the subject can be trained to operate the controller and control the tests themselves.

The images provided to each eye can be changed in one or more of, or possibly all of: content, colour, resolution, contrast, intensity, focus organisation and visual angle. Control of this change may be under the control of the user and/or the operator. This may stimulate and/or compensate the subjects vision, and be used to diagnose or treat a particular subject's condition. In addition, the image displayed to either eye can be selectively turned on or off (e.g. "patched"), or progressively fogged. The images displayed to each eye can be transposed/swapped over.

The subject may able be control the changes to the images.

The changes made may be to the whole image or just to a part of the image, for example the change may be in the peripheral field of view or in the central field of view. These changes can be made to vary the stimulus to the subject.

The ocular display apparatus can also be used to exercise the eyes and improve vision, for example by encouraging use of a 'lazy eye' in children with amblyopia. This may be achieved by showing less detail to the non-lazy eye, and details of more interest to the lazy eye, thereby making it work harder. The apparatus may comprise apparatus for measuring the type of and/or degree of physical performance of an eye, or eyes.

The requirement to use both eyes together stimulates binocularity, that is, the ability to use both eyes as a pair. This is in contrast to conventional patching treatment used for amblyopia sufferers which disrupts binocularity, and indeed may even result in a marked reduction in binocularly responsive cells in the brain, and the failure of the development of normal neurophysiological mechanisms, thus so-called 'normal' binocular vision may be impaired by patching. Indeed, in some circumstances, patching can induce amblyopia in what was the good eye.

The use of both eyes during treatment, diagnosis or exercise also maintains para-central fusion of the image with the good eye, even though the poorer eye may be more stimulated than the good. In addition, the use of both eyes encourages peripheral sensory fusion and aids motor fusion in the long term.

The ocular display apparatus described may be used to perform a range of orthoptic assessment tests, and to measure and diagnose ocular disorders, indeed the apparatus may even be used to exercise the eyes.

The apparatus may allow many tests to be performed, some of which may be complex tests. It may be a relatively small piece of equipment. The assessment procedure may be quicker and simpler, and may remove the need for subjective specialist expertise in the tests which requires extensive training and experience.

The simplicity of the apparatus means a technician could operate the apparatus, without requiring the input from a qualified orthoptist. Interpretation of results may however require more specialised medical input, such as from an orthoptist.

The apparatus described is more subject friendly than previous orthoptic test devices or apparatus, and in particular is useable with children as well as adults. Many conventional tests are too complicated or laborious for children.

The apparatus may also offer the opportunity to include tests for clinically important conditions for which there is currently no test available. This includes the assessment of paediatric visual fields, distance stereopsis, and post-operative diplopia test for cyclo-deviations.

Current methods for the diagnosis of amblyopia and other eye defects requires the subject to undertake a battery of orthoptic tests, to study vision, binocularity and ocular motility. This requires highly trained medical staff, a host of equipment - each with its own expensive acquisition, setting up and maintenance costs, and demands substantial space to set up and use the equipment. Furthermore, the extent and number of the tests can be somewhat daunting for a child.

It is also envisaged that the apparatus may be used as a pre-operative tool capable of simulating the vision a person will experience post-operation, such as for the correction of a squint. In some circumstances corrective surgery results in double vision, which may be considered by the person to be worse than the current condition, by testing preoperatively, this device may be able to predict which patient will suffer diplopia when their squint is corrected, including cyclo-deviations. In these circumstances it may be advisable not to operate. It also gives patients an opportunity to prepare themselves for the change in vision gradually. Current techniques require the injection of a Botulinum toxin which allows temporary adjustment of the eye position. This is, of course, invasive, and it is not pleasant to have an injection into an eye muscle.

An embodiment of the apparatus produces data in an electronic form, which can be easily stored, or transmitted to where needed. For example, a High Street optometrist could perform the test and then relay the data to the patient's GP for referral to the hospital eye service.

The apparatus may also be used to stimulate 3-dimensional vision by maintaining binocularity during treatment of amblyopia, the mechanisms involved in fusion are not disrupted. This may allow some development of stereoscopic vision not normally possible with conventional patching treatment.

Known visual devices, including Virtual Reality devices, deliver the same image or virtual environment to each eye. It is known to present different perspectives of the same scene to each eye in order to achieve a 3-D effect, but that is not the same as having "missing" objects for an eye - i.e. putting some of the interesting things a subject is intended to see to one eye only.

Software has been developed to provide treatment in the form of tests, quizzes, competitions, interactive video games, film clips and cartoons, which offers familiar and interactive presentations which children can actively enjoy whilst simultaneously improving their sight. Providing a performance to be watched, or an interactive event, such as a mental test/game, is psychologically more attractive to a patient than undergoing more traditional methods. By making the treatment more enjoyable, compliance should improve.

According to another aspect of the invention a data carrier carrying software, which when running on a processor, causes the processor tocontrol the display of a first image to one eye only of a subject, and a second, different, image to the subject's other eye only, the first and second images being presented to the subject so that they perceive a composite image, characterised in that the first image comprises a first sub-image and the second image comprises a second sub-image, the sub-images when superimposed produce a composite image, wherein
the first sub-image and the second sub-image each have a common component superimposable as seen by a subject so as to be seen as only one sub-image by the subject, and in which at least one of the sub-images also has at least one additional feature not present in the other sub-image,
the additional feature arranged to be presented to the eye that performs worst/badly in order to treat amblyopia; and
wherein at least one of the first or second images includes a moving object in addition to stationary objects.

According to a further aspect the invention provides a computer program product configured, for use with ocular display apparatus, to control a computer comprising means for displaying a first image to one eye only of a subject, and a second, different, image to the subject's other eye only, the first and second images being presented to the subject so that they perceive a composite image, characterised in that the first image comprises a first sub-image and the second image comprises a second sub-image, the sub-images when superimposed produce a composite image, wherein
the first sub-image and the second sub-image each have a common component superimposable as seen by a subject so as to be seen as only one sub-image by the subject, and in which at least one of the sub-images also has at least one additional feature not present in the other sub-image,
the additional feature arranged to be presented to the eye that performs worst/badly in order to treat amblyopia; and
wherein at least one of the first or second images includes a moving object in addition to stationary objects.

Embodiments of the invention will now be described in more detail by way of example with reference to the accompanying drawings, of which:-
**Figure 1** shows schematically an ocular/image display apparatus for use in studying visual function and ocular motility;
**Figure 2** shows the images visible to each eye in Figure 1 superimposed;
**Figure 3A** to **3C** show schematically an alternative ocular/image display apparatus to that of Figure 1;
**Figure 4** shows schematically a portable ocular/image display apparatus;
**Figure 5A** shows an ocular/image display apparatus configured as a headset;
**Figure 5B** shows a handheld ocular/image display apparatus;
**Figure 6** shows a computer control screen/interface used to operate the image display device of Figures 1, 3 to 3C, 5 and 5B;
**Figure 7** shows an enlarged view of part of the computer control screen/interface of Figure 6;
**Figures 8A to 8C** show details of a Pac Man™ type game designed for use with the ocular/image display apparatus of Figures 1, 3A to 3C, 5A and 5B;
**Figures 9A to 9C** show details of a racing car game designed for use with the ocular/image display apparatus of Figures 1, 3A to 3C 5A and 5B;
**Figures 10A to 10C** show an example of images used to view a film with the ocular/image display apparatus of Figures 1, 3A to 3C, 5A and 5B;
**Figures 11A to 11C** show images used with ocular/image display apparatus of Figures 1, 3A to 3C, 5A and 5B to study fusion range;
**Figures 12A to 12C** show images used with ocular/image display apparatus of Figures 1, 3A to 3C, 5A and 5B to study peripheral vision;
**Figures 13** **and** **14** show graphically the results of trials in which children with amblyopia have used the ocular display apparatus according to the invention.

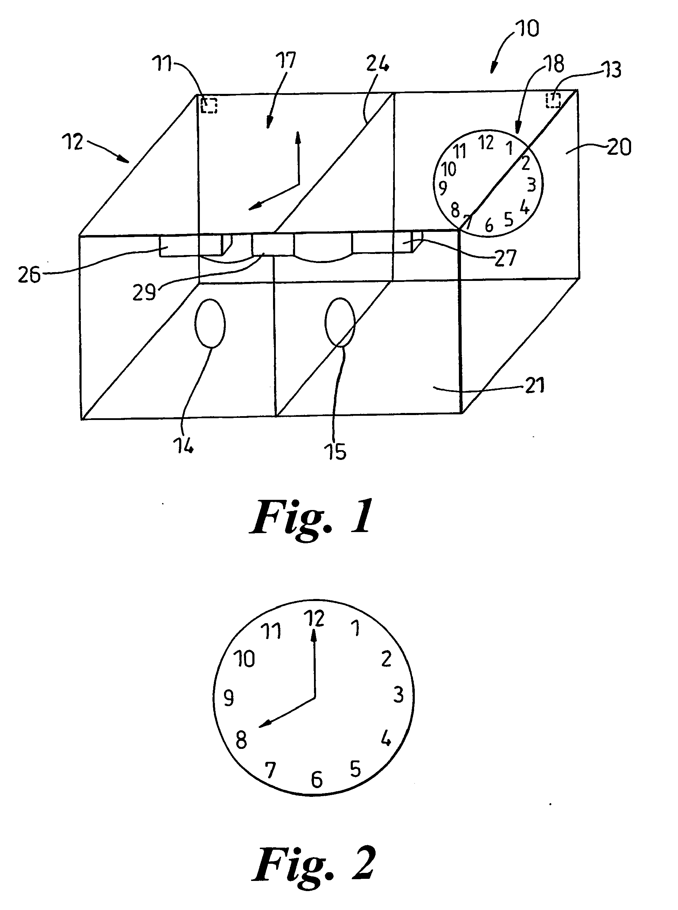

Figure 1 shows a schematic representation of an ocular or image display apparatus 10, configured to present images of differing visual content simultaneously to each eye. The apparatus 10 comprises a housing 12, into which a subject looks using eye holes 14, 15 in housing wall 21 to view images 17 and 18 displayed onto a screen on the opposite wall 20. Each eye views, using the eyeholes 14 and 15, different displayed images, 17 and 18 respectively. A central dividing wall 24 prevents each eye from seeing the image displayed to the other eye.

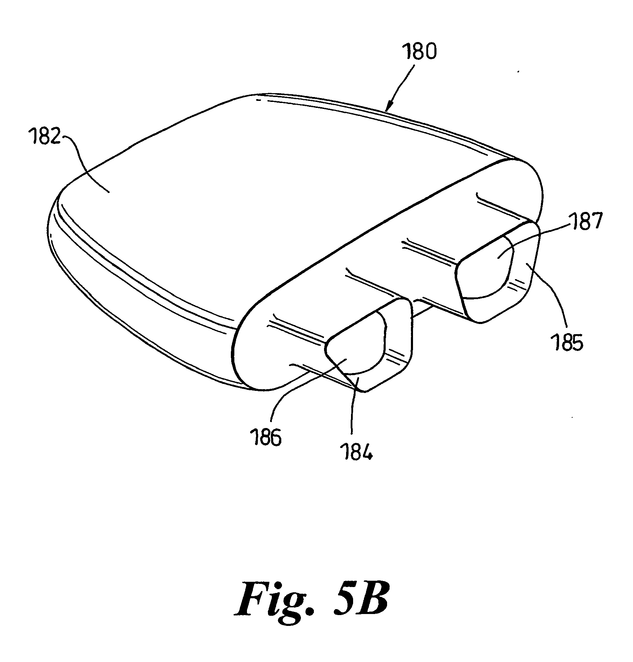

The eye holes 14, 15 may include eyepieces (not shown) similar to those illustrated in Figure 5B which serve to ensure the subject is visually immersed in the displayed images, and reduce the risk of distraction by events occurring in the subjects peripheral vision outside the displayed images.

The housing 12 is shown in Figure 1 as being transparent to allow the internal configuration of the apparatus 10 to be illustrated, however in use the housing will typically be opaque to prevent interference from external visual stimuli.

In Figure 1 small projectors 26 and 27 within the apparatus project images 17 and 18 onto the back wall or screen 20 of the device. The projectors 26, 27 are controlled by the controller 29.

In the simple test depicted in Figure 1 the subject is required to position the two displayed images 17 and 18, one being seen by each eye, such that they are superimposed and only one image is perceived. For example, the left eye looking through eye hole 14 will see a first image 17 of clock hands, and the right eye, looking through eye hole 15, will see a second image 18 of a clock face, the dividing wall 24 prevents the left eye from seeing the clock face 18 and the right eye from seeing the clock hands 17. The object of the test is for the subject to perceive the images superimposed, as a composite image, that is the clock hands on the clock face, as depicted in Figure 2. Once the images are aligned and superimposed the hands may move requiring the subject to maintain the image alignment in order to tell the time from the composite image. Furthermore, the subject must use both eyes together in order to tell the time. If only one eye is used the subject will be able to see only the hands or only the face and will not be able to tell the time.

Depending on the subjects visual/ocular characteristics alignment and superimposition of the first and second images may require the position or angle of the images to be adjusted. Typically the images are displayed along the visual axis of each of the subject's eyes.

The display of images 17 and 18 are controlled by controller 29. The controller 29 is configured to respond to the image perceived by the subject, and to adjust the location of the first and second images accordingly, until the subject perceives the composite image of the hands on the clock face as in Figure 2. The ability to adjust the angle of the image viewed allows a subject with a squint to use both eyes together and to resolve the image of the hands on the clock face.

Information may be fed to the controller manually, in this example in response to verbal fed back from the subject as to what he can actually see. Say, for example, the subject reports that he can see the entire clock face 18 however only the tip of one hand 17 is touching the edge of the clock face, this information can then be fed to the controller to effect movement of the projected image of the hands 17 horizontally until the subject perceives the clock hands on the clock face, thereby allowing subjective measurements to be taken.

Alternatively, sophisticated eye tracking devices 11, 13 may be incorporated into the ocular/image display apparatus, which monitor and electronically communicate to the controller 29 eye movements, more specifically, the direction in which each eye is looking, and hence what is being perceived by the subject. The operator can then adjust the image displayed accordingly until the subject perceives both images fully aligned and the hands are on the clock face.

By analysing the degree and nature of movement of the displayed images required in order for the subject to perceive images in the correct registration (the hands on the clock face), conclusions can be drawn as to the subject's visual function and ocular motility. This information may be displayed on an associated screen or print out.

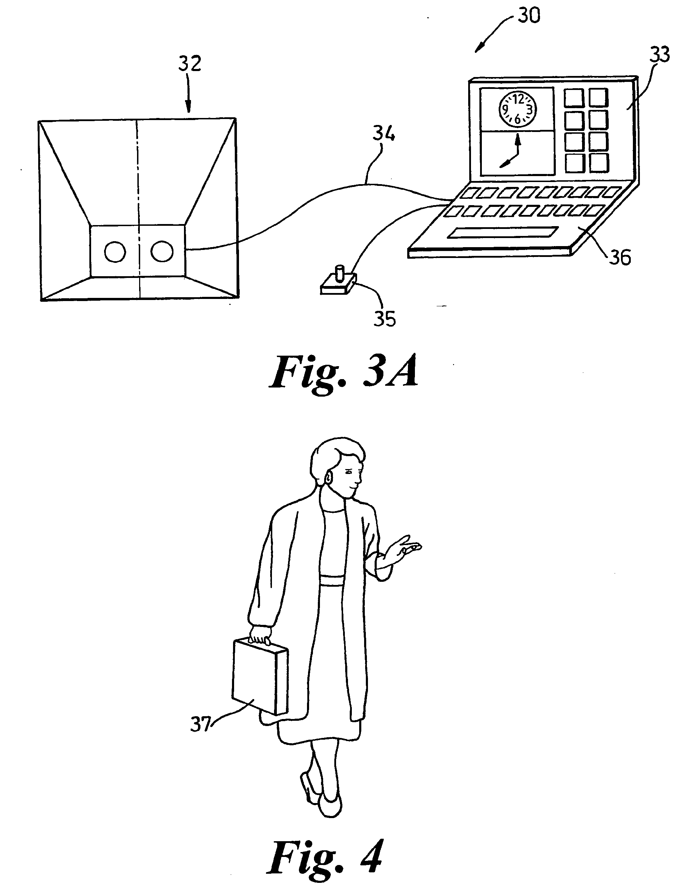

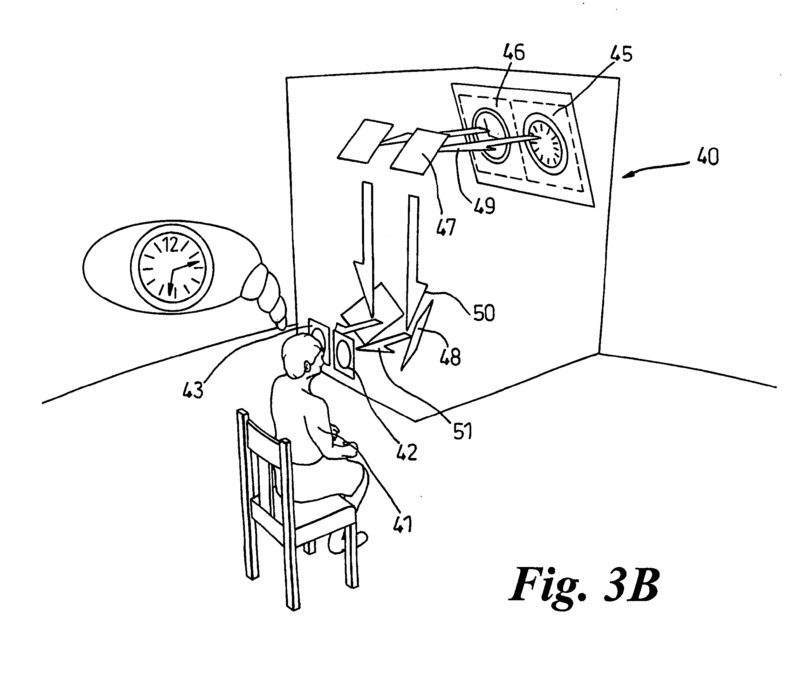

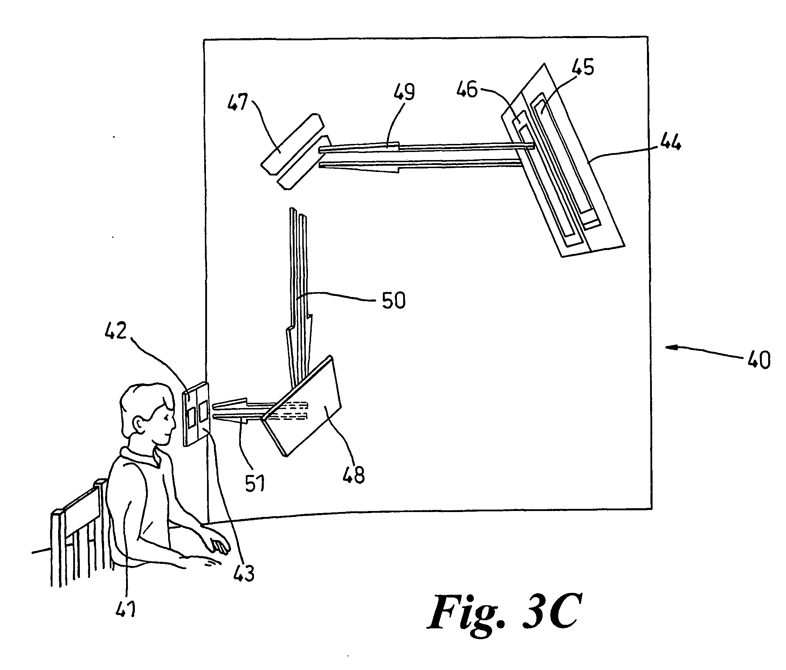

Figure 3A depicts an alternative ocular/image display apparatus 30 to that of Figures 1, 3B and 3C. In this representation the apparatus 30 comprises a remote viewer 32 connected to a controller, in this case a computer 33. The viewer 32 may be hard-wired 34 to the computer 33 or communicate via an infra red connection (not shown). Again the apparatus could be fitted with eyepieces as depicted in Figure 5B in order to encourage visual immersion.
An operator using the computer 33 can control and analyse the images seen by the subject in the viewer 32. Indeed, it may be that the subject or the operator can use a joystick 35, a keyboard 36 or another suitable device, to move the projected images until the desired superimposition of images is perceived. The computer will monitor, and possibly record, the extent of movement of the images.

Figure 3B shows an alternative viewer to that depicted in Figure 3A. The subject 41 is shown seated before the viewer 40. Using eye holes 42 and 43 the subject 41 sees a different image 45 and 46 with each eye. Again, the eyepiece of Figure 5B could be fitted to the viewer. Images 45, 46 are initially displayed, rotated 90°, on a screen 44 located to the rear of the viewer 40, a series of mirrors reflects the images 47, 48 from the screen so that they can be viewed in the correct orientation by the subject 41. Arrows 49, 50 and 51 indicate how image 45 is reflected. Figure 3C is an alternative view of the viewer 40 depicted in Figure 3B.

Figure 4 depicts a portable ocular/image display apparatus 37, which can be used at any location. This has the advantage that a subject need not travel to a large hospital in order to use the apparatus 37, indeed the apparatus could be used in the subjects own home, in a GP's clinic, or in a high street opticians. The results of tests could be e-mailed to the hospital/consultant.

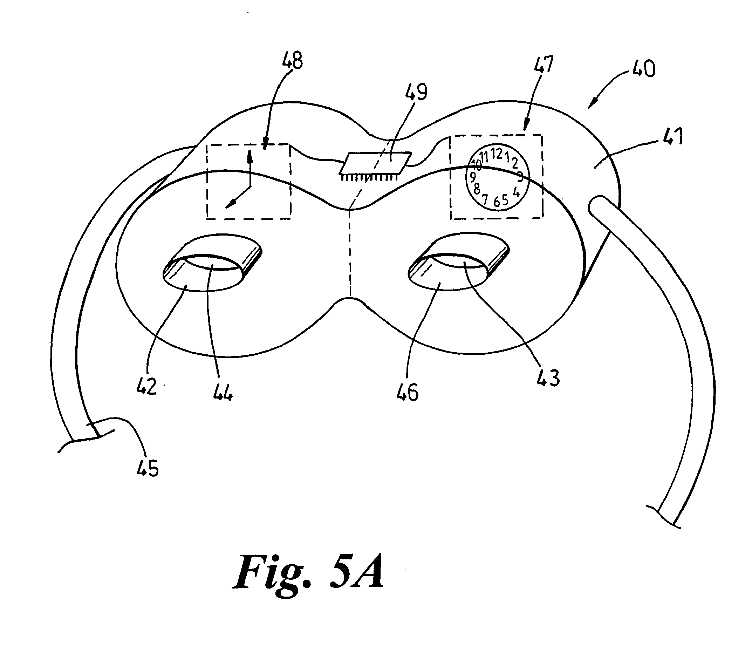

Figure 5A depicts a further alternative ocular/image projecting apparatus 40 to that of Figures 1 and 3A to 3C, configured as a headset to be worn by a subject. The apparatus comprises a pair of goggles 41, with lenses 43, 44, which is located on a subjects head using strap 45. Eyepieces 42, 46 project from the lenses 44, 43 and allow the subject to be substantially visually immersed in the displayed images when wearing the apparatus. Once positioned on the subjects head each eye is simultaneously shown a different projected image 47, 48, typically using two miniature computer display screens in the headset. Again, the images shown are of clock hands 48 and a clock face 47 and the aim is for the subject to locate the images such that the hands are perceived to be on the clock face as shown in Figure 2. Display of the images within the headset is controlled by the controller 49. The controller may be in communication with a remote computer which relays information regarding what images should be displayed and any movement of the images required.

Figure 5B depicts a yet further alternative ocular/image projecting apparatus 180 to that of Figures 1, 3A to 3C and 5A, configured to be hand held by the subject. The apparatus comprises a casing 182 which houses the display means (not shown) configured to display a different image to each eye of the subject. The subject locates the eyepieces 184, 185 around their eyes and then looks through lenses 186, 187 to view the displayed images.

Eyepieces 184, 185 typically contact the subject's face around the eye, thereby reducing the risk of visual distraction by activity in the peripheral field of vision outside the apparatus. Essentially the eyepieces 184, 185 help to ensure that the subject is visually immersed in the displayed images. This allows the apparatus to be used to examine and exercise the whole of the subjects visual field.

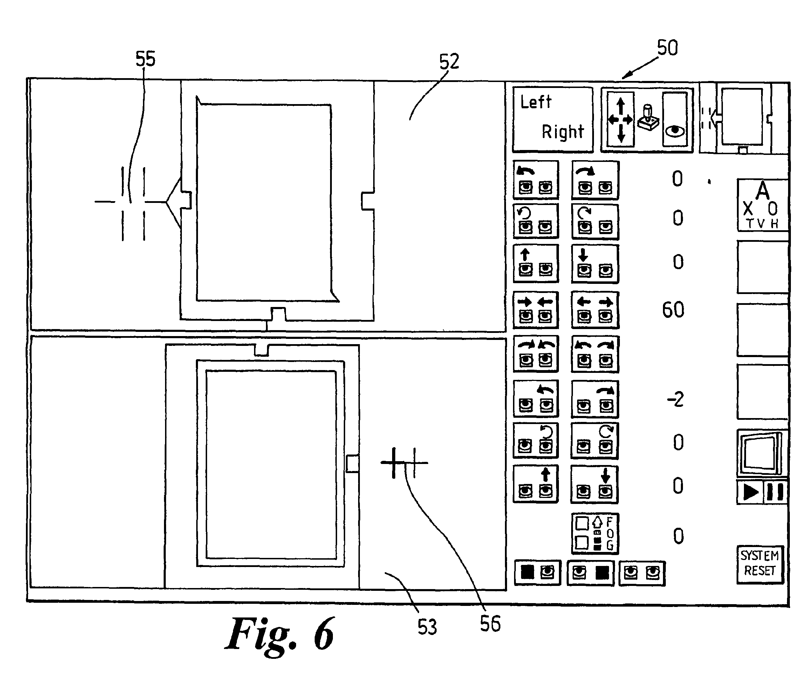

Figure 6 shows an example of a computer control screen/interface or operator display 50 used by an operator to control the images displayed in an associated ocular/image display apparatus, such as illustrated in Figures 1, 3A to 3C, 5A and 5B, to enable an assessment of the subjects visual function and ocular mobility. The operator may be a clinician, orthoptist or technician, or may even be the subject. The control screen/interface 54 includes a representation of the different images being shown to each eye. In this example, one eye is seeing the upper image 52 and the other eye is seeing the lower image 53. The images when seen by the subject are orientated to be horizontal. The aim is to position the images such that the subject perceives them to be superimposed. In this example an aerial is the "combined" aligned object and the subject has to align the object alignment portions 55, 56 such that the subject perceives a complete aerial. This control screen/interface 54 allows the operator to see what the subject is seeing.

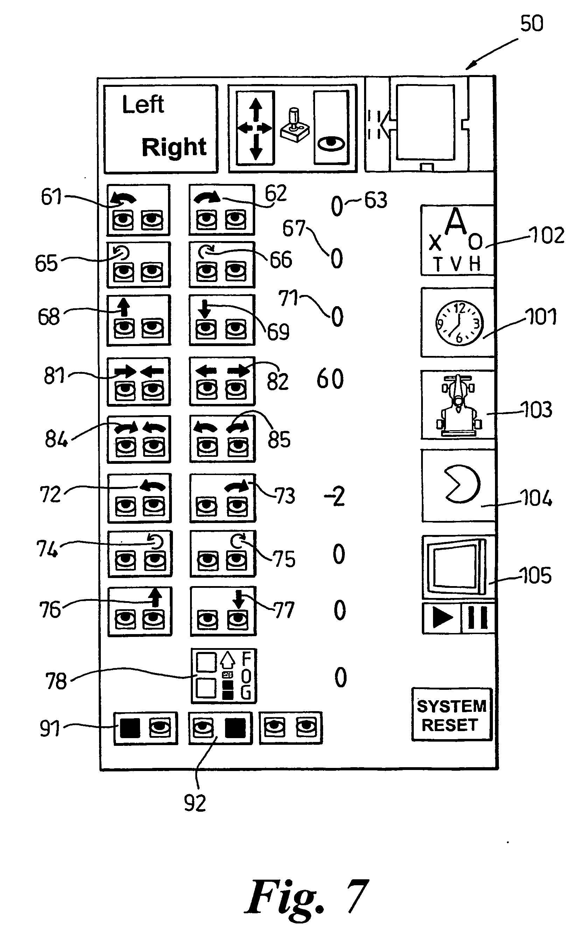

Figure 7 shows an enlarged view of part of the computer control screen/interface or operator depicted in Figure 6. The control panel or manipulation monitor 50 includes a series of buttons, typically operated using a mouse/cursor, control keys on a keyboard, or touch sensitive screen, which allow the position of the projected image to be adjusted to compensate for particular eye/visual conditions. The control panel allows various adjustments to be made to the displayed images, such as adjustment of the viewing angle to each eye, either independently or together, adjustment of the effective intra-ocular distance of the images and the selective display of images to either the good eye or the lazy eye or both eyes.

For example, buttons 61, 62 allow the image presented to the left eye to be adjusted anticlockwise and clockwise respectively to compensate for rotation of the eye. The degree of rotation is given as numeric indicator 63. In this example, the image displayed to the left eye has not been rotated, and a reading of 0 is seen.

Buttons 65 and 66 allow the image presented to the left eye to be rotated to the left and right respectively to compensate for torsion of the left eye, the degree of torsion is given as numeric indicator 67.

Buttons 68 and 69 allow the image presented to the left eye to be adjusted vertically, up and down respectively, to compensate for any vertical misalignment. The degree of movement is given as numeric indicator 71.

Buttons 72 to 77 reflect adjustment of the image with respect to the right eye for those considerations discussed with reference to buttons 61, 62, 65, 66, 68 and 69 respectively.

Buttons 81 and 82 allow the images to be simultaneously moved horizontally, button 81 will cause the images to be moved closer together, and button 82 will move the images further apart. This allows correction for any latent or manifest horizontal squint as well as allowing studies of convergence and divergence.

Buttons 84 and 85 allow images to be simultaneously rotated. Button 84 will rotate both images 'inwards', that is the image to the left eye will be rotated clockwise and the image to the right eye will be rotated anti-clockwise. Conversely, button 85 will rotate both images 'outwards', that is the image to the left eye will be rotated anti-clockwise, and the image to the right eye will be rotated clockwise.

There are also options to fog one of the images seen by the subject, button 88, and to patch one eye, buttons 91 and 92 patch the right and left eye respectively.

Ultimately, at the end of a session, the computer will produce a report, in this case a numerical readout, of the visual characteristics of the subject, such as the degree of squint or torsion in an eye, and the ability of the subject to converge, this data can be stored digitally or printed out as a paper copy. The data provided can be used to assist in diagnosis.

The control panel 50 also includes options to switch between different images and modes of use, that is, say between test, watch and game modes. Button 101 would select the clock face and hands images depicted in Figure 1. Button 102 would select a traditional logMAR visual acuity chart. Button 103 would select a car racing game and button 104 would select a Pac Man™ type game. Button 105 would select a film option. In all of the above options each eye would be simultaneously shown a different image.

The switch may be made by the subject or the operator, if that is not the subject.

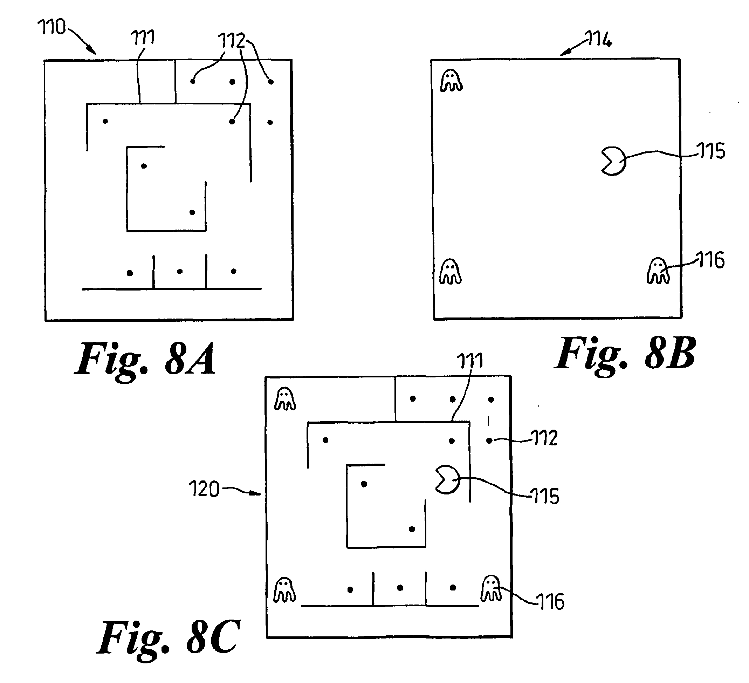

Figures 8A to 8C show a Pac Man™ type game designed for use with the ocular/image display apparatus of Figures 1, 3A to 3C and 5. A different image is simultaneously displayed to each eye, requiring both eyes to work together in order for the game to be played. The game requires subject interaction and participation.

In more detail, image 110 (Figure 8A) is shown to only one eye and includes the maze 111 and 'dots' 112 which when eaten by the Pac Man™ 115 results in the scoring of points. Simultaneously, image 114 (Figure 8B) is shown to the other eye. This image 114 includes the Pac Man™ 115 and the 'ghosts' 116. When the images are correctly projected such that the subject perceives both images 110 and 115, one with each eye, accurately superimposed, the subject will see image 120 (Figure 8C). Once the subject sees the image as 120 the game can be played, the aim being to negotiate the Pac Man™ 115 around the maze 111 eating as many dots 112 as possible, whilst avoiding the ghosts 116 who will destroy Pac Man™ 115. The game requires that both eyes are used simultaneously, and can be used as treatment for amblyopia. The playing of the game requires active participation by the subject, this interaction is preferably real-time, in that the game responds immediately to the subjects request, eg to move the Pac Man™ to the left or right. The game may be used as an alternative to patching or in combination with patching. Such exercises are more effective than patching as the subject, usually a child, is more interested in playing a game and thus compliance is more likely. In addition, this exercise requires both eyes to function as a pair, encouraging binocularity, as well as treating the amblyopia by forcing use of the weaker lazy eye.

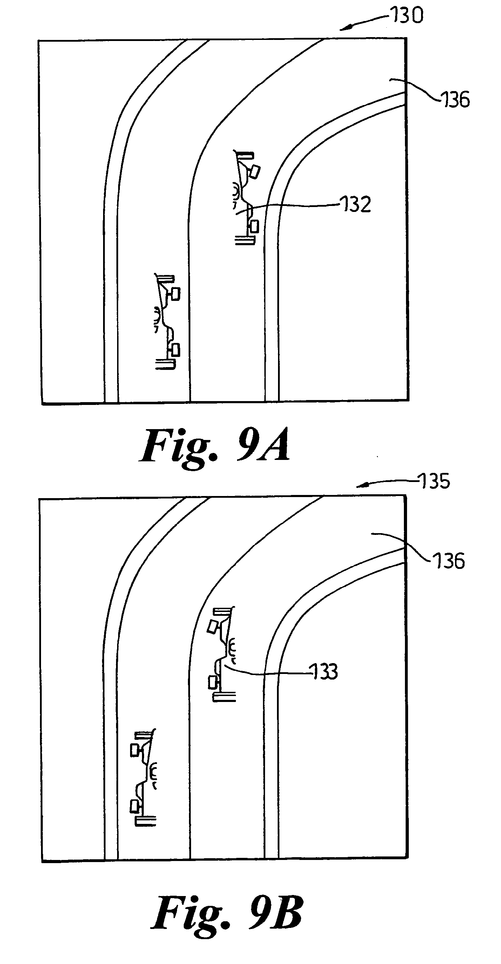

Figures 9A to 9C depict an alternative game to that shown in Figures 8A to 8C, in which the subject races a car 138 around a circuit/race track 136. Again, the game requires subject interaction and participation, a different image is simultaneously displayed to each eye and requires both eyes to work together in order for the game to be played.

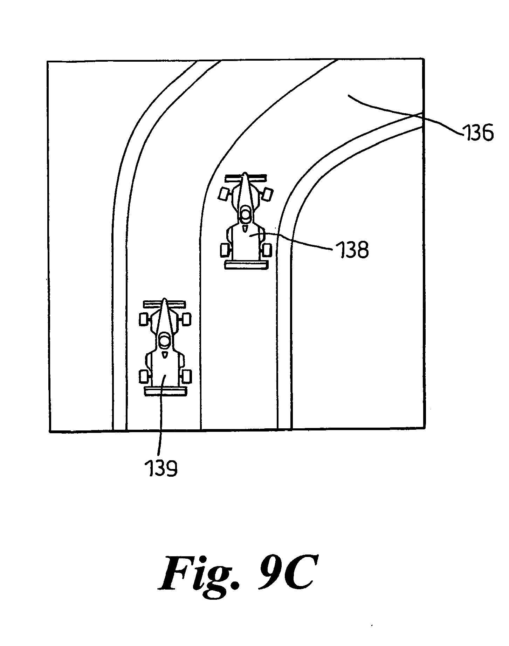

One eye is shown an image 130 (Figure 9A) showing the background racetrack 136 and the right hand half 132 of the racing cars one of the racing cars being under the subjects control. Simultaneously, the other eye is shown a different image 135 (Figure 9B), again showing the background racetrack 136 but this time the left hand half 133 of the racing cars is depicted. When both eyes are working together, and the images are accurately positioned, the subject perceives complete racing cars 138, 139 on the race track 136, as depicted in Figure 9C. The subject is then able to race their car, say 138, against the computer, or another player. Again, this game requires user participation and is designed to encourage both eyes to work together and to encourage binocularity.

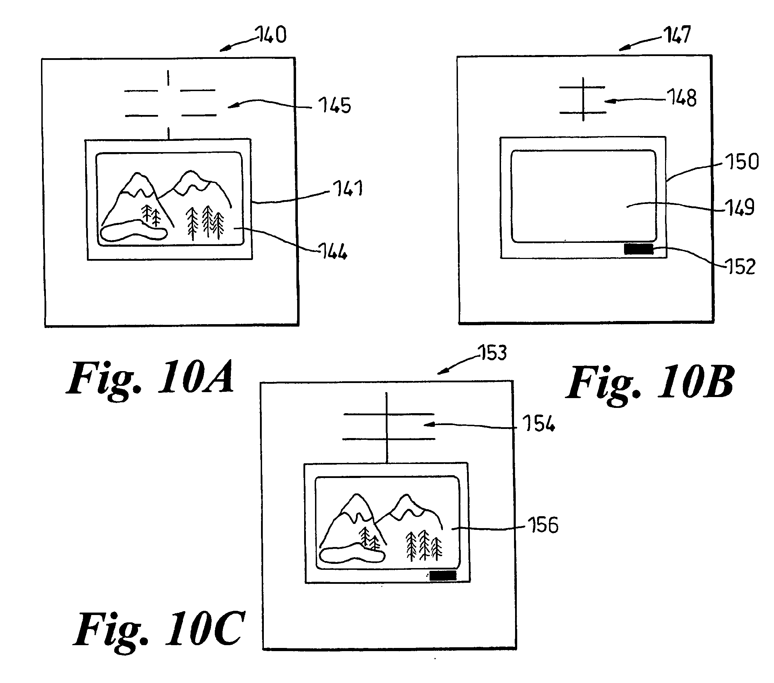

Figures 10A to 10C depict an alternative use of the image projecting device configured to allow the subject to watch a film, cartoons or the television. Again, each eye simultaneously sees different images.

More specifically, the good eye will typically be shown a static image, such as of television set 150 with a blank screen 149 (Figure 10B) . The lazy eye, on the other hand, will be shown a moving image 140 of a television set 141 turned on, with moving images being located on the static screen 144 (Figure 10A), such as, a film, cartoon or television programme. When the images are correctly positioned for the subject, the subject will perceive both screens accurately superimposed 156 (Figure 10C) and can watch the film. To ensure that the subject is using both eyes other peripheral features may be included, the presence of which (when a patient is questioned by a clinician), will confirm that both eyes are being used. For example, image 140 includes parts of an aerial 145, the central component 148 of which is depicted in image 147. When both eyes are used together and images 140 and 147 are superimposed a complete aerial 154 is perceived. Alternatively, image 147, projected to the good eye includes a block 152 the presence of which, while the film is being watched confirms that the subject is using both eyes e.g. the block can be coloured, for example red, and the patient could be asked "What colour is the light on the television?". If they do not know, they are not using the equipment properly, and the clinician knows they have to correct the way the subject is using the equipment and/or check it is working properly. By having "checks" or "tell-tales" or controls in what is observed the clinician can ensure that the subject is seeing what they are supposed to see.

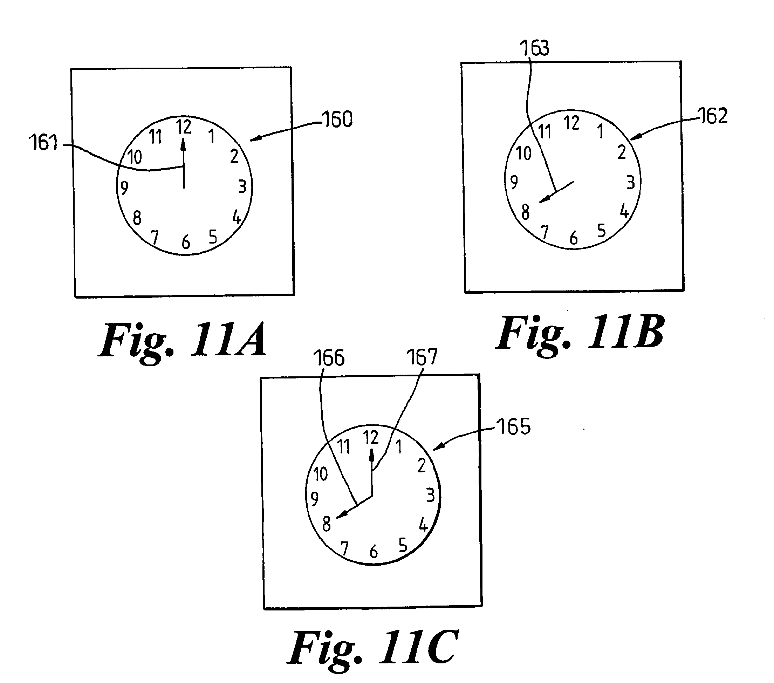

Figures 11A to 11C show an alternative test that can be carried out using the ocular display apparatus of this invention, in which there is not necessarily movement in the displayed images. In this case, one eye is shown an image of a clock 160 with only an hour hand 161 Figure 11A, and the other eye is shown an image of the same clock 162 with a minute hand 163 Figure 11B, when correctly aligned the subject perceives a clock 165 with a minute hand 166 and an hour hand 167 Figure 11C, and the subject can tell the time. This test can be used to measure fusion ranges, that is the combined of convergence and divergence range.

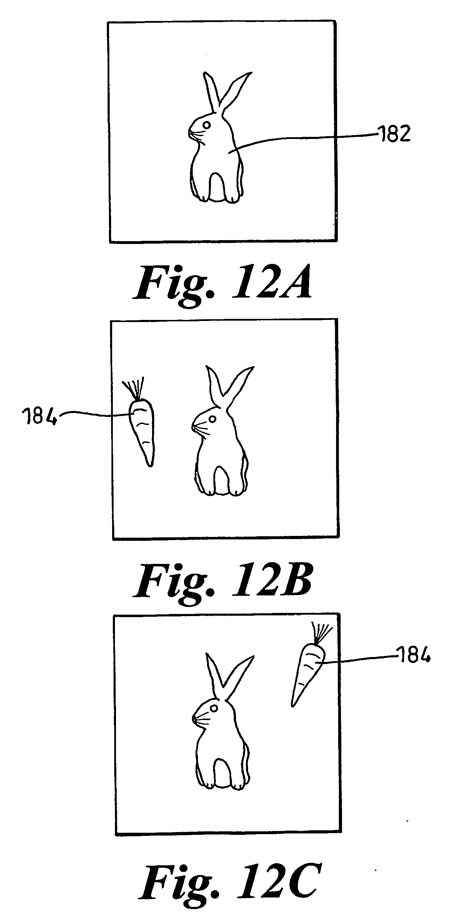

Figures 12A to 12C show an alternative test that can be carried out using the ocular/image display apparatus of this invention to study a subject's peripheral vision. The image of a rabbit 182 shown in Figure 12A is shown to both eyes and is located in the centre of the subject's visual field. The images may have to be aligned to ensure that only one rabbit is seen. Typically this object, in this case a rabbit 182, moves or changes colour in order to retain the subjects attention. Additional objects, in this example carrots 184, are then introduced into the image in the subjects peripheral field of view, as illustrated in Figures 12B and 12C. The images may be introduced in both or only one image, thereby testing one or both eyes. The subject is asked to communicate when they see an object in their peripheral vision, this may be verbally or electronically, say by pressing a keypad. By analysing when a subject does and does not see the additional objects a map of the subjects peripheral field of view can be created.

The ocular/image display apparatus can be used to perform a number of orthoptic assessment functions including:
**Measurement of vision** - for example using the latest logMAR acuity charts;
**Measuring 'crowding' effec**t - for example using modified logMAR acuity charts. Crowding causes reduced vision in some eye conditions, resulting in the inability to discriminate closely packed letters;
**Visual perception time** - measure the speed at which an object is seen (e.g. a letter of a certain size is seen). The letter (or object) could be shown to one eye only, or different intensity letters could be shown to the different eyes so that one is made to work harder than the other;
**Measurement of squint in primary position (straight ahead) or ocular misalignment** - important if surgery is to be undertaken as it may help to determine the amount of surgery required;
**Measurement of squint in nine (or several) positions of gaze** - to understand squints in which the deviation is not the same in all positions of gaze, currently this requires the use of the synoptophore or prism cover tests which is very laborious;
**Measurement of ocular torsion** - in the primary position and other gaze positions - torsion is measured in degrees and is a common symptom of subjects with diplopia which has a torsional element (cyclotorsion);
**Pictorial representation, or 'mapping', of eye movements** - using a modified Lees screen, for example: useful for subjects with complex eye movement disorders;
**Mapping area of binocular single vision** - that is the area in which a subject sees singular binocular vision, this is usually determined using a Goldmann field analyser;
**Mapping out the limit of eye movements** - particularly useful for subjects with severely restricted ocular movement;
**Measurement of density of suppression** - reduced levels of suppression can indicate that occlusion may cause intractable diplopia. Suppression is currently measured with the Sbisa bar comprising coloured filters of increasing density;
**Measurement of area of suppression** - usually done using a **synoptophore;**
**Measurement of abnormal head posture** - some subjects control a squint be moving their head, there is currently no easy way to measure this, however a headset could be used to measure head posture in degrees (e.g. the headset could have a gyroscope/other sensor, or it could be imaged and the image of the user's head plus headset/monitor device analysed to determine head position;
**Measurement of saccades** (rapid eye movements) - both horizontal and vertical saccades could be measured. Currently this is determined by asking a subject to look between two pens held in their eye line at either side of their head. We can project images and objectively measure eye movement/speed, as compared with subjective assessments;
**Post-operative diplopia test**, including torsional squints- performed to ascertain the risk of intractable diplopia following the correction of a squint, the test in normally performed with prisms, however distortion induced by larger prisms can introduce inaccuracies;

### Measurement of binocularity and paediatric visual field;

**Presence of binocular single vision** - determines how well the eyes work together, and is indicative of a squint and the level of control thereof. Potential binocularity can be determined in the angle of deviation is corrected first. Peripheral binocularity can be determined, for example, using Bagolini glasses in front of the eyepieces;
**Measurement of fusion range** - consists of determining the convergence and divergence range and allows the maintenance of binocularity to be determined;
**Measurement of 3-D vision** - can be determined by measuring stereo-acuity using stereotests;
**Measurement of distance stereopsis** - determined as for 3-D vision except the target is more distant;
**Paediatric visual field assessment** - when a person fixates straight ahead the amount of vision to the side is known as the visual field. Visual field defects can be indicative of problems with the visual pathway. Current tests such as the Humphrey field analyser are too difficult for most children and very time consuming testing a child's concentration span. Children are therefore generally tested using the confrontation method, which detects only gross defects. The ocular display apparatus according to the invention allows visual field to be simply and quickly plotted.

A protocol for studying visual fields in children, using the ocular/image display apparatus, comprises locating a small central target of interest to the child, such as a cartoon character, in the centre of the child's field of view. This figure could change or move to maintain interest. An eye-tracker (such as the ISCAN™) is set up to monitor the movement of one eye only and to confirm that the child is indeed looking at the central fixation target. Another visually stimulating image is then randomly presented in the periphery of the visual field. This target must be small enough to be peripheral but prominent enough to be readily seen, say, bright and high contrast. This appearance of the target is typically random with regards to when and where it appears in the visual field, and the central fixation target typically remains present throughout. The observer/computer records the appearance of a target image in the peripheral field and the subject's response thereto.

If the child sees the second target, in the periphery of the visual field, he will reflexly look at it. The eye tracker will register the saccadic (fast flick) eye movement of the child to see the second target and the computer/observer will register the target as seen, and record that part of the visual field is intact.

A series of second targets are typically presented in all four quadrants of the visual field. To ensure the test is short, simple and reliable a minimum number of targets will be used. In this way a simple map can be constructed of the child's field of view showing areas where it is intact, and areas where it is deficient.

In adults (or children), varying the intensity of the second targets to determine thresholds of visual field defects could refine the test.

In addition, the ocular/image display apparatus can also be used to treat visual condition, and to provide exercises, including:
**Treatment of amblyopia** - using exercises to encourage use of the lazy eye at the same time as the good eye. These exercises may comprise making the eyes look at different angles/positions on the display screen, with the "bad" eye shown the object of attention either more intensely/clearly than the "good" eye, or to the exclusion of the good eye, or such that only by using both eyes is the scene visible/comprehensible at all;
**Treatment of convergence insufficiency and reduced fusion** - by using programs to stimulate and exercise the muscles of the eye to increase the ability of the eyes to work together to 'fuse' objects are different distances where this ability is abnormally reduced;
**Orthoptic exercises** - the device can be used to perform exercises to improve convergence, fusion, or duction eye movements. An inability to converge can result in severe eye symptoms, and the ocular/image display apparatus can be used to present a binocular stimulus to encourage visual convergence. Conventional exercises are typically currently carried out for 2 to 5 minutes three to six times a day, it is envisaged that 10-15 minutes use of the image projection device will be of equal value. Duction exercises may also be undertaken, typically on subjects with dysthyroid eye disease. Duction exercises require a subject to follow a target from side to side or up and down depending on the direction of gaze that needs to be exercised. Exercises for subjects with reduced fusion and symptomatic eye strain/headaches could also be performed.

By way of example only, there follows the results of a study in which six children with amblyopia used ocular/image display apparatus according to the above described invention.

Six children under the age of eight were chosen for the study, all displayed various forms of amblyopia. The study required the children to attend the eye clinic regularly and to use the ocular display apparatus. The apparatus used was based upon that depicted in Figures 3B and 3C, configured such that a first image is displayed to one eye and a second, different, image is displayed to the other eye, wherein at least one image contains an object that moves. In order to perceive the complete image the children had to use both eyes.

Typically, the children would watch a film using the ocular display apparatus, the film being shown to the weaker lazy eye, however certain peripheral features in the image displayed to the good eye only were required to be seen, thereby ensuring both eyes were being used.

In addition, the children played a PacMan™ type game and a racing car game similar to those described in Figures 8A to 9C.

Each child made up to 12 visits to the clinic and used the ocular display apparatus for up to 300minutes (5 hours) in total. After each visit the childs vision was assessed using a standard LogMAR test.

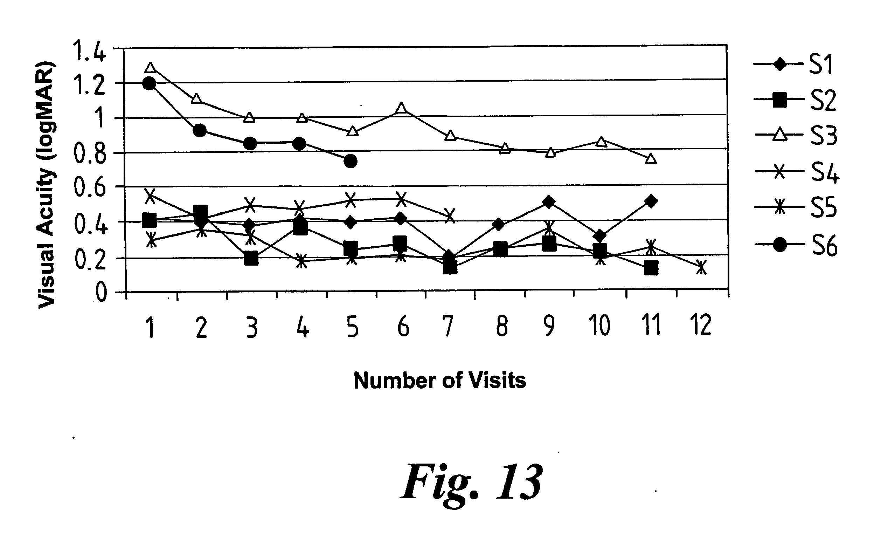

The change in the visual acuity in the amblyopic eye of the children after each visit was recorded and is presented graphically in Figure 13 (S1 to S6 are the children studied). A decrease in the visual acuity reading reflects an improvement in vision. The results demonstrate that five of the six children studied showed an improvement in vision in the amblyopic eye following use of the ocular display apparatus according to the invention.

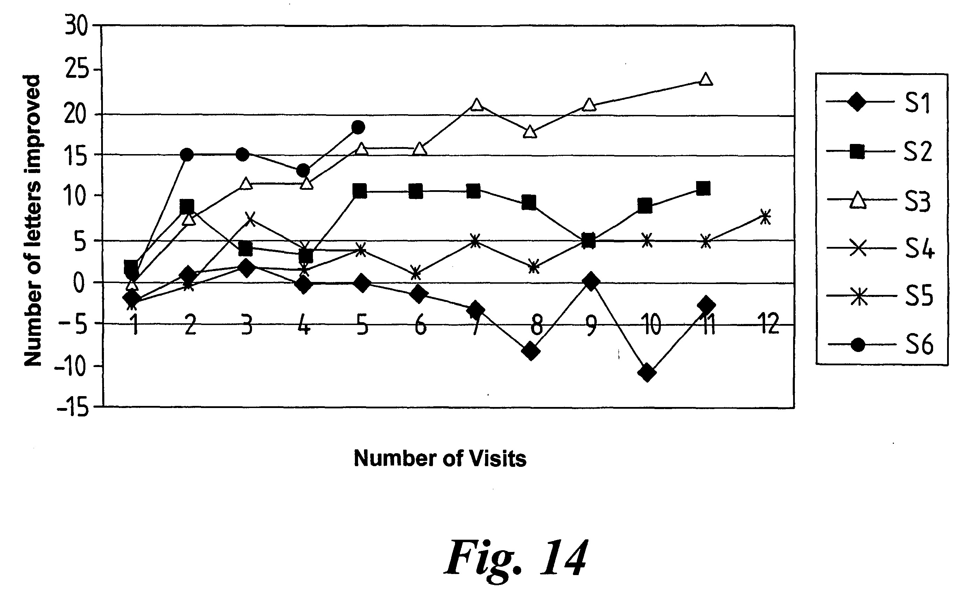

The change in overall vision of the children was also recorded after each visit and is presented graphically in Figure 14. The graph plots number of visits against the improvement in the number of letters of the LogMAR chart that each child could see. Again, 5 of the children showed an improvement in the number of letters they could see, in the best case an improvement from 5 to 24 letters was seen over the course of the study.

It can be seen from these results that marked improvements in the vision of children with amblyopia can be seen after less then 300 minutes using the ocular display apparatus of this invention. Conventional patching techniques can require 400 or more hours of treatment for similar results to be seen.

It may be helpful to review in more detail the known prior art.

In particular EP 0830839 discloses a device using a lenticular screen, which would not work if the subject were amblyopic. The angle of viewing a lenticular screen is also important in determining the location of the images as perceived by the viewer. This is not so with embodiments of the present invention. There is no discussion of using this device to treat visual disorders, nor does it envisage user control of movement as is required in the active game-playing mode of the subject application.

GB 2 353 869 A discloses a digital synoptophore and performs only the functions of a traditional synoptophore - no movement within the presented images is envisaged.

US 4 756 305 (Mateik) discloses an eye training device configured to display a different image to each eye, however the images displayed in US 4 756 305 are not truly dynamic, they are static LCD images which jump between alternative position - they are sultatory, rather than dynamic. The images in US 4 756 305 are viewed via a prism which introduces chromatic aberration and degrades the visual image, full colour images are not possible. There is no clinician control or remote monitoring in US 4 756 305. US 4 756 305 does not envisage a rapid change of the image viewed and can only display one image type, that is, you cannot readily change say between an eye test, a film and a game as allowed for in the subject application or to change the image content to one eye. Use of the device of US 4 756 305 to treat, measure or correct for a squint is not envisaged.

A non-limiting list of significant differences between the known prior art and some embodiments of the present invention include:
- visual immersion - in some embodiments of the present invention the subject has no significant peripheral view outside the displayed image so is not distracted, and the whole visual field can be studied;
- dynamic movement - in some embodiments of the present invention the whole visual field may move - what is moved, and to what extent, can be varied dependent on the subjects conditions i.e. the degree of amblyopia, also must be able to change the movement to ensure the subject is cognitively engaged;
- images are displayed in full colour in some embodiments of the present invention;
- images may be presented along the direction of any visual axis in some embodiments of the present invention;
- some embodiments of the present invention allow for interactive involvement of the user with the images, such as participation in a game;
- some embodiments of the present invention allow for operator monitoring/control of the images displayed;
- some embodiments of the present invention allow for control of the images, or parts of the images, which make up the peripheral and the central vision;
- some embodiments of the present invention allow for the ability to vary the stimulus, i.e. what is moved;
- some embodiments of the present invention allow for egocentric (first person) movement - so it appears that the subject (viewer) is actually moving; and
- the versatility of the device of some embodiments of the present invention allows the user or operator to readily switch between test, watch and game modes.

## Claims

1. Ocular display apparatus for the treatment of amblyopia, the apparatus having image presentation means adapted to display a first image (147) to one eye only of a subject, and a second, different, image to the subject's other eye only (140), the first and second images (140, 147) being presented to the subject so that they perceive a composite image, wherein the first image (147) comprises a first sub-image and the second image (140) comprises a second sub-image, the sub-images when superimposed produce a composite image (153), wherein
the first sub-image and the second sub-image each have a common component (141, 150) superimposable as seen by a subject so as to be seen as only one sub-image by the subject, and in which one of the sub-images also has at least one additional feature (144) not present in the other sub-image,
the additional feature (144) arranged to be presented to the eye that performs worst/badly in order to treat amblyopia; and
and **characterised in that** at least one of the first or second images includes a moving object in addition to stationary objects.

2. Apparatus according to claim 1 in which the subject is visually immersed in the displayed images.

3. Apparatus according to any preceding claim in which the images (140, 147) are computer generated.

4. Apparatus according to any preceding claim adapted to produce images (140, 147) which are perceived as two-dimensional, three-dimensional or virtual reality.

5. Apparatus according to any preceding claim in which the image presentation means includes one or more screens.

6. Apparatus according to any preceding claim in which the object movement appears to the subject as smooth.

7. Apparatus according to any preceding claim in which object movement is in the peripheral visual field, the central visual field or the whole visual field.

8. Apparatus according to any preceding claim in which the images (140, 147) are displayed and viewed in full colour.

9. Apparatus according to any preceding claim in which the images (140, 147) are presented along the subject's visual axis.

10. Apparatus according to any preceding claim arranged to allow the subject to perceive egocentric movement, in which the images (140, 147) are presented such that it appears to the subject that they are moving within the displayed composite image.

11. Apparatus according to any preceding claim which uses virtual reality technology.

12. Apparatus according to any preceding claim in which said first image (147) includes only stationary objects and said second image (140) includes at least one moving object, and which has an object-control, adapted to enable control of the movement of at least one of the moving objects.

13. Apparatus according to any preceding claim in which said first image (147) includes at least one first moving object and said second image (140) includes at least one second moving object, and which has an object-control adapted to enable control of the movement of at least one of, or both of, the first and/or second moving objects.

14. Apparatus according to claim 12 or claim 13 in which the object-control is manipulatable by the subject and/or by an operator and/or by software.

15. Apparatus according to any preceding claim comprising an operator display adapted to display to an operator the images (140, 147), or representation of the images (140, 147), seen by the subject.

16. Apparatus according to claim 15 adapted to display to an operator an indication of the type or degree of eye disorder present in the subject.

17. Apparatus according to any preceding claim comprising image manipulation means adapted to enable the first and second images to be manipulated to present them so that a subject perceives the intended composite image (153).

18. Apparatus according to claim 17 in which the manipulation means are provided to the subject or to the operator or to both.

19. Apparatus according to claim 17 or claim 18 comprising a manipulation monitor adapted to provide information on the type and degree of manipulation of the images required to enable the subject to perceive an intended composite image (153).

20. Apparatus according to any preceding claim adapted to present a composite image (153) that presents a game, or viewable performance, or subject-interactive test to the subject.

21. Apparatus according to claim 20 wherein a game requires the subject's interactive participation/interaction.

22. Apparatus according to claim 21 wherein the interaction is real-time.

23. Apparatus according to any preceding claim adapted to measure visual function.

24. Apparatus according to any preceding claim adapted to measure visual function and to present a game, viewable performance or subject interactive test to the subject.

25. Apparatus according to claim 24 wherein the subject or an operator can readily switch between test, watch or game modes.

26. Apparatus according to any preceding claim which is portable.

27. Apparatus according to any preceding claim comprising a device which monitors eye movement.

28. Apparatus according to any preceding claim comprising apparatus for exercising an eye, or eyes.

29. Apparatus according to any preceding claim arranged as a pre-operative tool to simulate the vision the subject will experience post operation.

30. Apparatus according to any preceding claim in which the first image (147) contains a first alignment portion (145) which when the subject perceives the intended composite image is aligned with a second alignment portion (148) in the second image (140).

31. A data carrier carrying software, which when running on a processor, causes the processor to control the display of a first image (147) to one eye only of a subject, and a second, different, image (140) to the subject's other eye only, the first and second images (140, 147) being presented to the subject so that they perceive a composite image (153), **characterised in that** the first image (147) comprises a first sub-image and the second image (140) comprises a second sub-image, the sub-images when superimposed produce a composite image (153), wherein
the first sub-image and the second sub-image each have a common component (141, 150) superimposable as seen by a subject so as to be seen as only one sub-image by the subject, and in which at least one of the sub-images also has at least one additional feature (144) not present in the other sub-image,
the additional feature (144) arranged to be presented to the eye that performs worst/badly in order to treat amblyopia; and
**characterised in that** at least one of the first or second images includes a moving object in addition to stationary objects.

32. A computer program product configured, for use with ocular display apparatus, to control a computer comprising means for displaying a first image (147) to one eye only of a subject, and a second, different, image (140) to the subject's other eye only, the first and second images (140, 147) being presented to the subject so that they perceive a composite image (153), **characterised in that** the first image (147) comprises a first sub-image and the second image (140) comprises a second sub-image, the sub-images when superimposed produce a composite image (153), wherein
the first sub-image and the second sub-image each have a common component (141, 150) superimposable as seen by a subject so as to be seen as only one sub-image by the subject, and in which at least one of the sub-images also has at least one additional feature (144) not present in the other sub-image,
the additional feature (144) arranged to be presented to the eye that performs worst/badly in order to treat amblyopia; and
**characterised in that** at least one of the first or second images includes a moving object in addition to stationary objects.

## Patentansprüche

1. Bildanzeigevorrichtung zur Behandlung von Sehschwäche, wobei der Apparat ein Hilfsmittel zur Bildpräsentation aufweist, das adaptiert wurde, um dem einen Auge einer Testperson ein erstes Bild (147) und nur dem anderen Auge der Testperson ein zweites, unterschiedliches Bild anzuzeigen, wobei das erste und das zweite Bild (140, 147) der Testperson präsentiert werden, sodass diese ein zusammengesetztes Bild wahrnimmt, wobei das erste Bild (147) ein erstes Teilbild und das zweite Bild (140) ein zweites Teilbild umfasst, und die Teilbilder - wenn sie überlagert werden - ein zusammengesetztes Bild (153) ergeben, worin
das erste Teilbild und das zweite Teilbild jeweils eine gemeinsame Komponente (141, 150) haben, welche überlagerbar ist, wie sie von einer Testperson gesehen wird, damit von der Testperson nur ein Teilbild wahrgenommen wird, und worin eines der Teilbilder auch mindestens ein zusätzliches Merkmal (144) aufweist, das im anderen Teilbild nicht vorhanden ist,
das zusätzliche Merkmal (144), das angeordnet ist, um dem Auge präsentiert zu werden, welches am schlechtesten/schlecht abschneidet, um eine Sehschwäche zu behandeln;
und **dadurch gekennzeichnet ist, dass** zumindest das erste und/oder das zweite Bild ein sich bewegendes Objekt zusätzlich zu unbeweglichen Objekten beinhaltet.

2. Vorrichtung gemäß Anspruch 1, in der die Testperson in die gezeigten Bilder visuell eintaucht.

3. Vorrichtung gemäß einem der vorhergehenden Ansprüche, in der die Bilder (140, 147) computergeneriert sind.

4. Vorrichtung gemäß einem der vorhergehenden Ansprüche, die adaptiert wurde, um Bilder (140, 147), die als zweidimensional, dreidimensional oder als Virtual Reality wahrgenommen werden, zu erzeugen.

5. Vorrichtung gemäß einem der vorhergehenden Ansprüche, in der das Hilfsmittel zur Bildpräsentation einen oder mehrere Bildschirme beinhaltet.

6. Vorrichtung gemäß einem der vorhergehenden Ansprüche, in der die Bewegung des Objekts der Testperson als gleichmäßig erscheint.

7. Vorrichtung gemäß einem der vorhergehenden Ansprüche, in der die Bewegung des Objekts im peripheren Gesichtsfeld, im zentralen Gesichtsfeld oder im gesamten Gesichtsfeld stattfindet.

8. Vorrichtung gemäß einem der vorhergehenden Ansprüche, in der die Bilder (140, 147) in Farbe angezeigt und betrachtet werden.

9. Vorrichtung gemäß einem der vorhergehenden Ansprüche, in der die Bilder (140, 147) entlang der Sehachse der Testperson präsentiert werden.

10. Vorrichtung gemäß einem der vorhergehenden Ansprüche, die entsprechend angeordnet ist, um der Testperson die Wahrnehmung einer egozentrischen Bewegung zu ermöglichen, in der die Bilder (140, 147) so präsentiert werden, dass es für die Testperson den Anschein hat, als ob sie sich innerhalb des angezeigten zusammengesetzten Bilds bewegen würde.

11. Vorrichtung gemäß einem der vorhergehenden Ansprüche, in der Virtual-Reality-Technologie verwendet wird.

12. Vorrichtung gemäß einem der vorhergehenden Ansprüche, in der das erste Bild (147) nur unbewegliche Objekte beinhaltet und das zweite Bild (140) mindestens ein bewegliches Objekt beinhaltet, wobei die Vorrichtung eine Objektsteuerung umfasst, die adaptiert wurde, um die Steuerung der Bewegung von mindestens einem der sich bewegenden Objekte zu ermöglichen.

13. Vorrichtung gemäß einem der vorhergehenden Ansprüche, in der das erste Bild (147) mindestens ein erstes sich bewegendes Objekt beinhaltet und das zweite Bild (140) mindestens ein zweites sich bewegendes Objekt beinhaltet, und die eine Objektsteuerung aufweist, die adaptiert wurde, um die Steuerung der Bewegung von mindestens einem oder beiden der ersten und/oder zweiten sich bewegenden Objekte zu steuern.

14. Vorrichtung gemäß Anspruch 12 oder Anspruch 13, in der die Objektsteuerung durch die Testperson und/oder einen Betreiber und/oder Software manipulierbar ist.

15. Vorrichtung gemäß einem der vorhergehenden Ansprüche, die ein Betreiber-Display umfasst, das adaptiert wurde, um einem Betreiber die Bilder (140, 147) oder eine Darstellung der Bilder (140, 147), die von der Testperson gesehen werden, zu zeigen.

16. Vorrichtung gemäß Anspruch 15, die adaptiert wurde, um einem Betreiber einen Hinweis auf die Art oder den Grad der bei der Testperson vorliegenden Sehstörung zu geben.

17. Vorrichtung gemäß einem der vorhergehenden Ansprüche, die Hilfsmittel zur Bildmanipulation beinhaltet, die adaptiert wurden, damit das erste und zweite Bild manipuliert werden können, um sie so zu präsentieren, dass eine Testperson das beabsichtigte zusammengesetzte Bild (153) wahrnimmt.

18. Vorrichtung gemäß Anspruch 17, in der die Hilfsmittel zur Manipulation der Testperson oder dem Betreiber oder beiden zur Verfügung gestellt werden.

19. Vorrichtung gemäß Anspruch 17 oder Anspruch 18, die einen Manipulationsmonitor umfasst, welcher adaptiert wurde, um Informationen zur Art und zum Grad der Bildmanipulation zu liefern, welche erforderlich ist, damit die Testperson in der Lage ist, ein beabsichtigtes zusammengesetztes Bild (153) wahrzunehmen.

20. Vorrichtung gemäß einem der vorhergehenden Ansprüche, die adaptiert wurde, um ein zusammengesetztes Bild (153) darzustellen, das ein Spiel, eine sichtbare Vorführung oder einen interaktiven Test für die Testperson präsentiert.

21. Vorrichtung gemäß Anspruch 20, worin ein Spiel die interaktive Teilnahme/Interaktion der Testperson verlangt.

22. Vorrichtung gemäß Anspruch 21, worin die Interaktion in Echtzeit ist.

23. Vorrichtung gemäß einem der vorhergehenden Ansprüche, die adaptiert wurde, um die Sehfunktion zu messen.

24. Vorrichtung gemäß einem der vorhergehenden Ansprüche, die adaptiert wurde, um die Sehfunktion zu messen, und der Testperson ein Spiel, eine sichtbare Vorführung oder einen interaktiven Test zu präsentieren.

25. Vorrichtung gemäß Anspruch 24, worin die Testperson oder ein Betreiber leicht zwischen dem Test-, Betrachtungs- oder Spielemodus hin- und herwechseln können.

26. Vorrichtung gemäß einem der vorhergehenden Ansprüche, die tragbar ist.

27. Vorrichtung gemäß einem der vorhergehenden Ansprüche, die ein Gerät umfasst, das die Augenbewegung überwacht.

28. Vorrichtung gemäß einem der vorhergehenden Ansprüche, die einen Apparat beinhaltet, um Übungen mit dem Auge oder den Augen durchzuführen.

29. Vorrichtung gemäß einem der vorhergehenden Ansprüche, die als präoperatives Instrument angelegt ist, um das Sehvermögen der Testperson nach der Operation zu simulieren.

30. Vorrichtung gemäß einem der vorhergehenden Ansprüche, in der das erste Bild (147) einen ersten Anordnungsabschnitt (145) enthält, welcher mit einem zweiten Anordnungsabschnitt (148) im zweiten Bild (140) angeordnet ist, wenn die Testperson das beabsichtigte zusammengesetzte Bild wahrnimmt.

31. Eine datentragende Software, die, wenn sie auf einem Prozessor läuft, dazu führt, dass der Prozessor die Anzeige eines ersten Bildes (147) nur für das eine Auge der Testperson und ein zweites, unterschiedliches Bild (140) für das andere Auge der Testperson steuert, wobei das erste und das zweite Bild (140, 147) der Testperson präsentiert werden, sodass diese ein zusammengesetztes Bild (153) wahrnimmt, was **dadurch gekennzeichnet ist, dass** das erste Bild (147) ein erstes Teilbild und das zweite Bild (140) ein zweites Teilbild umfasst, und die Teilbilder ein zusammengesetztes Bild (153) ergeben, wenn sie überlagert werden, worin
das erste Teilbild und das zweite Teilbild jeweils eine gemeinsame Komponente (141, 150) haben, welche überlagerbar ist, wie sie von einer Testperson gesehen wird, damit von der Testperson nur ein Teilbild wahrgenommen wird, und worin mindestens eines der Teilbilder auch mindestens ein zusätzliches Merkmal (144) aufweist, das im anderen Teilbild nicht vorhanden ist,
das zusätzliche Merkmal (144), das angeordnet wurde, um dem Auge, das am schlechtesten/schlecht abschneidet, präsentiert zu werden, um eine Sehschwäche zu behandeln; und
**dadurch gekennzeichnet ist, dass** zumindest das erste und/oder das zweite Bild ein sich bewegendes Objekt zusätzlich zu unbeweglichen Objekten beinhaltet.

32. Ein Computerprogrammprodukt, das zur Verwendung mit einer Bildanzeigevorrichtung konfiguriert ist, um einen Computer zu steuern, der ein Hilfsmittel zur Anzeige eines ersten Bildes (147) nur für das eine Auge der Testperson und ein zweites, unterschiedliches Bild (140) für das andere Auge der Testperson umfasst, wobei das erste und das zweite Bild (140, 147) der Testperson so präsentiert werden, dass diese ein zusammengesetztes Bild (153) wahrnimmt, was **dadurch gekennzeichnet ist, dass** das erste Bild (147) ein erstes Teilbild und das zweite Bild (140) ein zweites Teilbild umfasst, und die Teilbilder ein zusammengesetztes Bild (153) ergeben, wenn sie überlagert werden, worin
das erste Teilbild und das zweite Teilbild jeweils eine gemeinsame Komponente (141, 150) haben, welche überlagerbar ist, wie sie von einer Testperson gesehen wird, damit von der Testperson nur ein Teilbild wahrgenommen wird, und worin eines der Teilbilder auch mindestens ein zusätzliches Merkmal (144) aufweist, das im anderen Teilbild nicht vorhanden ist,
das zusätzliche Merkmal (144), das angeordnet ist, um dem Auge präsentiert zu werden, welches am schlechtesten/schlecht abschneidet, um eine Sehschwäche zu behandeln;
und **dadurch gekennzeichnet ist, dass** zumindest das erste und/oder das zweite Bild ein sich bewegendes Objekt zusätzlich zu unbeweglichen Objekten beinhaltet.

## Revendications

1. Un appareil de visualisation oculaire pour le traitement de l'amblyopie, l'appareil possédant un moyen de présentation d'images adapté de façon à montrer une première image (147) à un oeil uniquement d'un sujet, et une deuxième image, différente, à l'autre oeil uniquement du sujet (140), les première et deuxième images (140, 147) étant présentées au sujet de sorte qu'il perçoive une image composite, où la première image (147) comprend une première sous-image et la deuxième image (140) comprend une deuxième sous-image, les sous-images lorsqu'elles sont superposées produisant une image composite (153), où
la première sous-image et la deuxième sous-image possèdent chacune une composante commune (141, 150) superposable telle que vue par un sujet de façon à être vue comme n'étant qu'une seule sous-image par le sujet, et dans laquelle une des sous-images possède également au moins une caractéristique additionnelle (144) non présente dans l'autre sous-image,
la caractéristique additionnelle (144) étant agencée de façon à être présentée à l'oeil qui perçoit mal/le plus mal afin de traiter l'amblyopie, et
**caractérisé en ce qu'**au moins une de la première ou de la deuxième image comprend un objet mobile en plus d'objets stationnaires.

2. Un appareil selon la Revendication 1 dans lequel le sujet est visuellement immergé dans les images montrées.

3. Un appareil selon l'une quelconque des Revendications précédentes dans lequel les images (140, 147) sont générées par ordinateur.

4. Un appareil selon l'une quelconque des Revendications précédentes adapté de façon à produire des images (140, 147) qui sont perçues comme étant bidimensionnelles, tridimensionnelles ou de réalité virtuelle.

5. Un appareil selon l'une quelconque des Revendications précédentes dans lequel le moyen de présentation d'images comprend un ou plusieurs écrans.

6. Un appareil selon l'une quelconque des Revendications précédentes dans lequel le déplacement d'objet apparaît au sujet comme étant régulier.

7. Un appareil selon l'une quelconque des Revendications précédentes dans lequel le déplacement d'objet se situe dans le champ visuel périphérique, le champ visuel central ou la totalité du champ visuel.

8. Un appareil selon l'une quelconque des Revendications précédentes dans lequel les images (140, 147) sont montrées et visionnées en couleurs.

9. Un appareil selon l'une quelconque des Revendications précédentes dans lequel les images (140, 147) sont présentées dans l'axe visuel du sujet.

10. Un appareil selon l'une quelconque des Revendications précédentes agencé de façon à permettre au sujet de percevoir un déplacement égocentrique, dans lequel les images (140, 147) sont présentées de sorte qu'il semble au sujet qu'elles se déplacent à l'intérieur de l'image composite montrée.

11. Un appareil selon l'une quelconque des Revendications précédentes qui utilise une technologie de réalité virtuelle.

12. Un appareil selon l'une quelconque des Revendications précédentes dans lequel ladite première image (147) comprend uniquement des objets stationnaires et ladite deuxième image (140) comprend au moins un objet mobile, et qui possède une commande d'objet adaptée de façon à permettre la commande du déplacement d'au moins un des objets mobiles.

13. Un appareil selon l'une quelconque des Revendications précédentes dans lequel ladite première image (147) comprend au moins un premier objet mobile et ladite deuxième image (140) comprend au moins un deuxième objet mobile, et qui possède une commande d'objet adaptée de façon à permettre la commande du déplacement d'au moins un des, ou des deux, premier et/ou deuxième objets mobiles.

14. Un appareil selon la Revendication 12 ou 13 dans lequel la commande d'objet est manipulable par le sujet et/ou par un opérateur et/ou par un logiciel.

15. Un appareil selon l'une quelconque des Revendications précédentes comprenant un écran d'opérateur adapté de façon à montrer à un opérateur les images (140, 147), ou une représentation des images (140, 147), vues par le sujet.

16. Un appareil selon la Revendication 15 adapté de façon à montrer à un opérateur une indication du type ou degré de trouble oculaire présent chez le sujet.

17. Un appareil selon l'une quelconque des Revendications précédentes comprenant un moyen de manipulation d'images adapté de façon à permettre aux première et deuxième images d'être manipulées de façon à les présenter de sorte qu'un sujet perçoive l'image composite voulue (153).

18. Un appareil selon la Revendication 17 dans lequel le moyen de manipulation est fourni au sujet ou à l'opérateur ou aux deux.

19. Un appareil selon la Revendication 17 ou 18 comprenant un moniteur de manipulation adapté de façon à fournir des informations relatives au type et degré de manipulation des images requis pour permettre au sujet de percevoir une image composite voulue (153).

20. Un appareil selon l'une quelconque des Revendications précédentes adapté de façon à présenter une image composite (153) qui présente un jeu, ou une prestation visionnable, ou un test sujet interactif au sujet.

21. Un appareil selon la Revendication 20 où un jeu exige la participation/interaction interactive du sujet.

22. Un appareil selon la Revendication 21 où l'interaction est en temps réel.

23. Un appareil selon l'une quelconque des Revendications précédentes adapté de façon à mesurer une fonction visuelle.

24. Un appareil selon l'une quelconque des Revendications précédentes adapté de façon à mesurer une fonction visuelle et à présenter un jeu, une prestation visionnable ou un test sujet interactif au sujet.

25. Un appareil selon la Revendication 24 où le sujet ou un opérateur peut aisément commuter entre les modes test, visionnage ou jeu.

26. Un appareil selon l'une quelconque des Revendications précédentes qui est portatif.

27. Un appareil selon l'une quelconque des Revendications précédentes comprenant un dispositif qui surveille un mouvement oculaire.

28. Un appareil selon l'une quelconque des Revendications précédentes comprenant un appareil destiné à exercer un oeil ou les yeux.

29. Un appareil selon l'une quelconque des Revendications précédentes agencé sous la forme d'un outil pré-opératoire destiné à simuler la vision que le sujet connaîtra après l'opération chirurgicale.

30. Un appareil selon l'une quelconque des Revendications précédentes dans lequel la première image (147) contient une première partie d'alignement (145) qui, lorsque le sujet perçoit l'image composite voulue est alignée avec une deuxième partie d'alignement (148) dans la deuxième image (140).

31. Un support de données contenant un logiciel, qui, lorsqu'il est exécuté sur un processeur, amène le processeur à commander la présentation d'une première image (147) à un oeil uniquement d'un sujet et une deuxième image, différente, (140) à l'autre oeil uniquement du sujet, les première et deuxième images (140, 147) étant présentées au sujet de sorte qu'il perçoive une image composite (153), **caractérisé en ce que** la première image (147) comprend une première sous-image et la deuxième image (140) comprend une deuxième sous-image, les sous-images lorsqu'elles sont superposées produisant une image composite (153), où
la première sous-image et la deuxième sous-image possèdent chacune une composante commune (141, 150) superposable telle que vue par un sujet de façon à être vue comme n'étant qu'une seule sous-image par le sujet, et dans laquelle au moins une des sous-images possède également au moins une caractéristique additionnelle (144) non présente dans l'autre sous-image,
la caractéristique additionnelle (144) étant agencée de façon à être présentée à l'oeil qui perçoit mal/le plus mal afin de traiter l'amblyopie, et
**caractérisé en ce qu'**au moins une de la première ou de la deuxième image comprend un objet mobile en plus d'objets stationnaires.

32. Un produit de programme informatique configuré, pour une utilisation avec un appareil de visualisation oculaire, de façon à commander un ordinateur comprenant un moyen destiné à montrer une première image (147) à un oeil uniquement d'un sujet, et une deuxième image, différente, (140) à l'autre oeil uniquement du sujet, les première et deuxième images (140, 147) étant présentées au sujet de sorte qu'il perçoive une image composite (153), **caractérisé en ce que** la première image (147) comprend une première sous-image et la deuxième image (140) comprend une deuxième sous-image, les sous-images lorsqu'elles sont superposées produisant une image composite (153), où
la première sous-image et la deuxième sous-image possèdent chacune une composante commune (141, 150) superposable telle que vue par un sujet de façon à être vue comme n'étant qu'une seule sous-image par le sujet, et dans laquelle au moins une des sous-images possède également au moins une caractéristique additionnelle (144) non présente dans l'autre sous-image,
la caractéristique additionnelle (144) étant agencée de façon à être présentée à l'oeil qui perçoit mal/le plus mal afin de traiter l'amblyopie, et
**caractérisé en ce qu'**au moins une de la première ou de la deuxième image comprend un objet mobile en plus d'objets stationnaires.
